Europäisches Patentamt

European Patent Office

Office européen des brevets

(11) Veröffentlichungsnummer : **0 130 351**
**B1**

(12) EUROPÄISCHE PATENTSCHRIFT

(45) Veröffentlichungstag der Patentschrift :
01.10.86

(51) Int. Cl.⁴ : **A 61 B 17/36**

(21) Anmeldenummer : 84105958.7

(22) Anmeldetag : 25.05.84

(54) Instrument zur indirekten Behandlung mit Wärme und Moxarauch.

(30) Priorität : 15.09.83 DE 3333271
25.06.83 EP 83106228

(43) Veröffentlichungstag der Anmeldung :
09.01.85 Patentblatt 85/02

(45) Bekanntmachung des Hinweises auf die Patenterteilung : 01.10.86 Patentblatt 86/40

(84) Benannte Vertragsstaaten :
AT BE CH DE FR GB IT LI LU NL SE

(56) Entgegenhaltungen :
FR-A- 624 952
US-A- 1 817 823
US-A- 4 203 438

(73) Patentinhaber : Nottbohm, Friedrich, Dr. Med.
Am Reisenbrook 24a
D-2000 Hamburg 67 (DE)

(72) Erfinder : Nottbohm, Friedrich, Dr. Med.
Am Reisenbrook 24a
D-2000 Hamburg 67 (DE)

(74) Vertreter : Fleck, Thomas, Dr.Dipl.-Chem. et al
Patentanwälte Raffay, Fleck & Partner Postfach 32 32
17
D-2000 Hamburg 13 (DE)

**Beschreibung**

Die Erfindung betrifft ein Instrument zur indirekten Behandlung mit Wärme und Moxarauch nach dem Oberbegriff des Hauptanspruchs. Ein derartiges Instrument ist aus der US-A-4 203 438 bekannt.

Ein ähnliches Instrument wird ferner von August Brodde in seinem Buch über « Brennen mit Moxakraut », WBV Biologisch-Medizinische Verlagsgesellschaft mbH & Co. KG, 1. Auflage, 1981, auf Seite 47, beschrieben. Dort wird das fernöstliche Verfahren des Anräucherns mit Moxakraut unter Verwendung einer Tabakspfeife zum Räuchern beschrieben. Das Mundstück ist danach entfernt und sein Ansatzstück am Pfeifenkopf mit Kerzenmaterial oder Glaserkitt verschlossen. In Richtung einer Diagonalen durch den Pfeifenkopf ist außen eine Bohrung angebracht. Nach dem dort beschriebenen Verfahren legt man eine Dosis Moxapulver in den dem Mundstück zugekehrten Winkel des Pfeifenkopfes, entzündet sie mit einem Räucherstäbchen und bläst nun mit gespitzten Lippen einen gerichteten Luftstrom über das glimmende Moxapulver, so daß er durch die Bohröffnung mit heißem Moxa-Rauch zusammen austritt. Hält man die Pfeife dann dicht an die Haut, entsteht ein intensives Wärmeempfinden, das man durch Verändern der Entfernung zwischen Pfeife und Haut variieren kann. Daß ein derartiges Instrument nicht nur primitiv im Aufbau, sondern auch in seiner Handhabung unbequem und umständlich ist, dürfte ohne weiteres einleuchten.

Der vorliegenden Erfindung liegt deshalb die Aufgabe zugrunde, das eingangs genannte Instrument derart zu verbessern, daß es einfach und schnell handhabbar ist und gleichzeitig ein möglichst hohes Maß an therapeutischer Effektivität bietet.

Ein weiteres Ziel der vorliegenden Erfindung ist es, die Wärme entsprechend der Hitzeverträglichkeit der Haut individuell steuern zu können, um so bei jedermann die für ihn optimale Ausnutzung der Rauchwirkung in der vorgegebenen Zeiteinheit zu erzielen. Außerdem soll das Instrument möglichst einfach und preiswert in der Herstellung sein, so daß eine Massenproduktion möglich ist.

Die vorstehenden Aufgaben und Ziele werden durch die im Anspruch 1 gekennzeichneten Merkmale gelöst.

Mit dem erfindungsgemäßen Instrument, das auch als Moxer bezeichnet werden kann, ist es möglich, den Rauch der Moxaglut in der gewünschten Weise auf die Haut zu richten. Dabei geht die Erfindung zunächst einmal vom Einsatz einer sog. Moxaziggare aus, die in der für die Therapie notwendigen Menge in die Rauchkammer einbringbar und nachschiebbar ist, wo sie ihre Rauchwirkung entfaltet. Dabei ist sie in Axialrichtung in der Räucherkammer auf- und abbeweglich, wodurch die Wärme wie gewünscht reguliert und die Glut im gewünschten Abstand von der Haut gehalten werden kann. Das Schutzgitter ist vorgesehen, damit die Asche vom Moxakraut nicht direkt auf die Haut fällt, sondern zurückgehalten wird.

Die Technik des Moxens sieht dabei folgendermaßen aus :

Das erfindungsgemäße Instrument wird mit seiner Kappe auf die Haut der zu behandelnden Person gehalten. Sofern möglich, kann diese dieses aufgrund des länglichen Stiels an vielen Stellen des Körpers das Instrument auch selbst applizieren. Durch das Schutzgitter der Kappe hindurch wird die Haut vom Rauch der Moxaglut angeräuchert, erwärmt. Über der Haut entsteht ein Hitzestau, der schon bald von der Haut nicht mehr toleriert wird. Die Hitzeverträglichkeit der Haut ist erreicht. Der Moxer kann nun leicht (1 bis 2 cm) angehoben und wieder auf die Haut abgesenkt werden. Hierbei kann der heiße Rauch verstärkt nach außen entweichen und die Haut toleriert dann so die Hitze wieder. Das erneute Absenken der Moxaziggare bzw. des erfindungsgemäßen Instrumentes auf die Haut läßt wieder einen Hitzestau entstehen, der nur unzureichend über die Rauchkammeröffnungen entweichen kann und erst bei leichtem Anheben des erfindungsgemäßen Instrumentes vollständig entweicht. Dieses leichte Anheben und Absenken des Moxers wird als Tupfen bezeichnet. Andererseits läßt sich natürlich die Hitze auf dadurch steuern, daß die Moxaglut durch Auf- oder Abbewegen des Stempels mehr oder weniger nahe an die Haut herangeführt wird. Wenn die Hitzeverträglichkeit immer nur ganz kurz überschritten wird, und man sich weitgehend im oberen Verträglichkeitsbereich aufhält, kann man eine optimale Ausnutzung der Rauchwirkung in der vorgegebenen Zeiteinheit erreichen. Läßt die Intensität der Hitzeentwicklung nach, in der Regel nach 3 bis 5 Minuten, muß die Asche vom Moxakraut vorsichtig abgestrichen werden, bis die Glut wieder zu sehen ist. Hierzu wird dann die Moxazigarre in Richtung weiter in die Kammer vorgeschoben. Mit anderen Worten, die paßgerechte Öffnung in der Rauchkammer ermöglicht erfindungsgemäß, daß der Abstand Moxaglut zum Schutzgitter nachreguliert werden kann.

Weitere Vorteile und Merkmale gehen aus den Unteransprüchen hervor.

Im folgenden wird ein bevorzugtes Ausführungsbeispiel der vorliegenden Erfindung näher anhand der vorliegenden Zeichnungen beschrieben. Es zeigt :

Figur 1 eine perspektivische Ansicht des erfindungsgemäßen Instruments ;

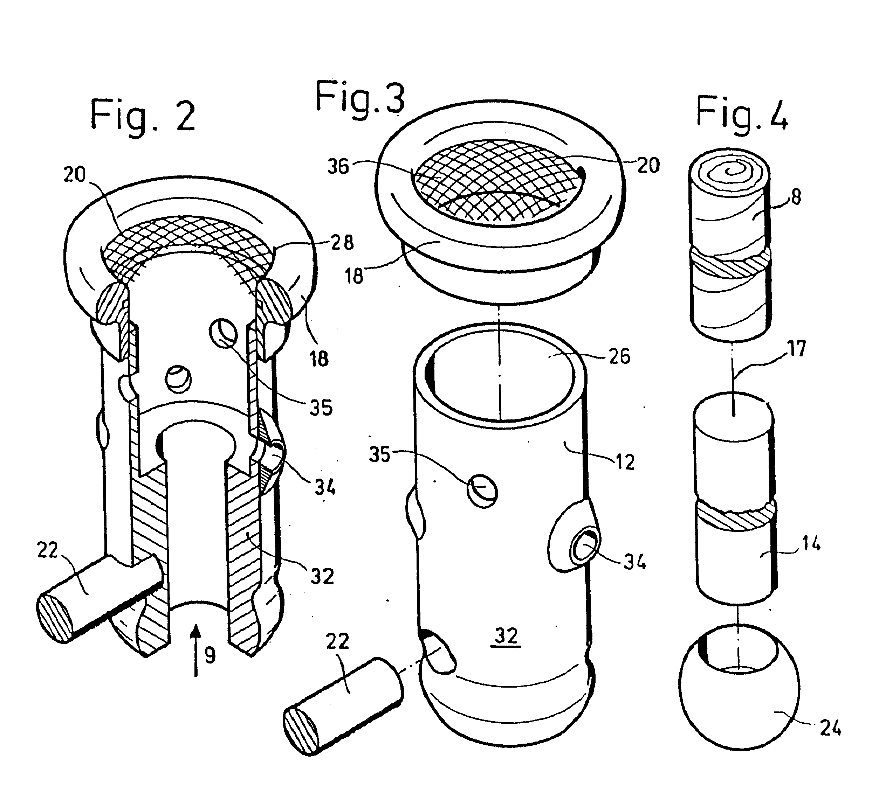

Figur 2 eine perspektivische Teilansicht des in Fig. 1 gezeigten erfindungsgemäßen Instrumentes, bei der die Rauchkammer teilweise im Schnitt dargestellt ist, um einen besseren Einblick in das Innere zu ermöglichen ; und

Figur 3 eine Explosionsdarstellung der dargestellten Ansicht ;

Figur 4 perspektivische Ansicht des losen Stempels und des Moxazigarrenhalters.

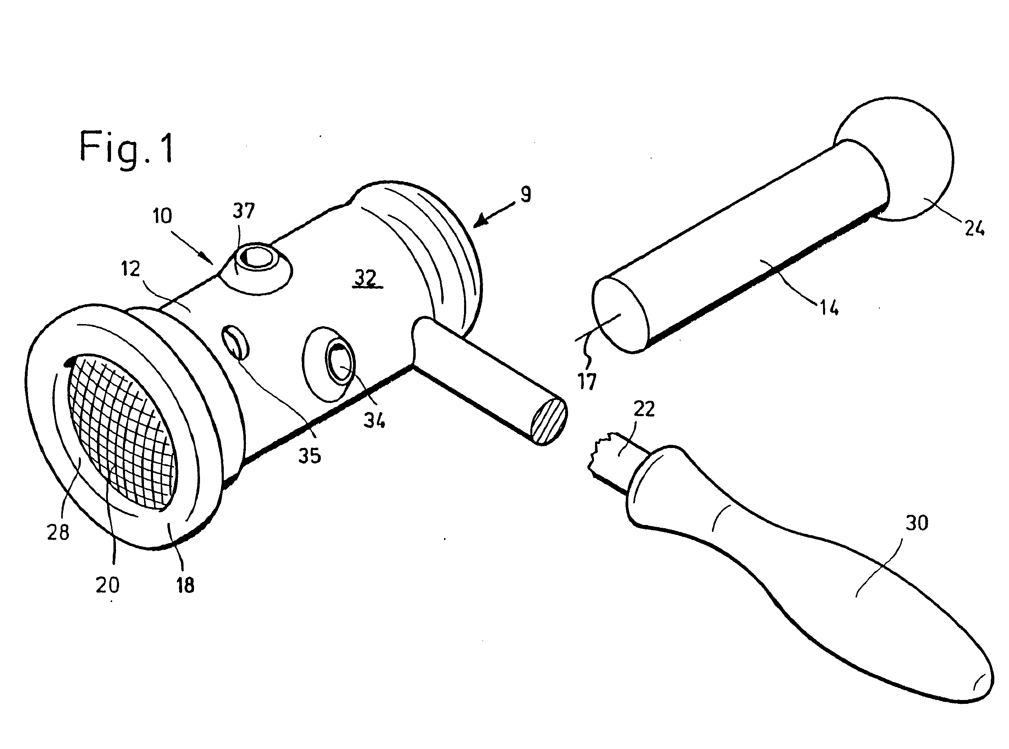

In Fig. 1 ist das erfindungsgemäße Instrument zur indirekten Behandlung mit Wärme und Moxarauch zur Selbsttherapie bei verschiedenen chronischen Schmerzproblemen allgemein mit 10 bezeichnet und auf der Seite liegend dargestellt. Es weist eine zylindrische Rauchkammer 12 auf, in der die Moxazigarre 8 im Gebrauchszustand durch die paßgerechte Öffnung 9 (nicht gezeigt) eingeschoben, sowie auf- und abbewegbar ist. Gegebenenfalls kann zum Nachschieben auch ein Stempel 14 verwendet werden, dessen eines Ende als Knauf 24 ausgebildet ist und dessen gegenüberliegendes Ende einen Dorn 17 aufweist. Der Benutzer des erfindungsgemäßen Instrumentes kann also einfach die angezündete Zigarre 8 in Axialrichtung in die Rauchkammer einschieben (mit oder ohne Hilfe des Stempels).

Die Figuren zeigen ferner deutlich, daß die Kappe 18 ein relativ engmaschiges Gitter bzw. ein Schutzgitter 20 aufweist. Um das erfindungsgemäße Instrument 10 überhaupt leicht und schnell handhaben zu können, d. h. es mit der brennenden Moxazigarre in der Rauchkammer 12 mit der Kappe 18 auf den gewünschten Hautbereich des Körpers aufzusetzen, ist ein länglicher Stiel 22 vorgesehen, der im an die Rauchkammer 12 angrenzenden Massivteil 32 eingelassen und befestigt ist. Das andere Ende des länglichen Stiels 22 weist einen Handgriff 30 auf.

Die Rauchkammer 12 ist mit der Umgebungsluft über Öffnungen 34, 35 verbunden, die den für die Verbrennung des Moxakrautes notwendigen Sauerstoff in die Rauchkammer 12 führen. Die Öffnungen 34 weisen jeweils außen einen sie umgebenden Vorsprung 37 auf, welcher einen Schornsteineffekt hervorruft und das Zugsystem verbessert.

In der vorliegenden Ausführungsform weisen die Rauchkammer 12, die Kappe 18 sowie die Austrittsöffnungen 26, 36 kreisförmige Struktur auf. Der Kappenrand 28 verläuft im vorliegenden Fall senkrecht. Jedoch ist in einer bevorzugten Ausgestaltung auch daran gedacht, daß dieser Kappenrand einen nach außen kegelförmigen Verlauf aufweisen kann. Das Schutzgitter 20 ist etwas beabstandet von der unteren Öffnung 26 der Rauchkammer 12 innenliegend im Kappenrand 28 eingelassen und besteht aus einem dünnen Metalldraht. Die Kappe 18 ist abnehmbar, um das Innere der Räucherkammer reinigen zu können und auch Asche von der Zigarre vorn zu entfernen. Im Gebrauch ist die Kappe 18 jedoch fest mit der Räucherkammer 12 verbunden.

Abschließend seien noch einige allgemeine Bemerkungen zum Moxakraut und Moxen mit der erfindungsgemäßen Vorrichtung gemacht :

Moxakraut ist das abgelagerte und getrocknete Kraut des gemeinen Beifußes (herba artemisae capillaris), das vorwiegend im asiatischen Raum angebaut wird. Der Einsatz von Moxakraut hat sich in der traditionellen chinesischen Medizin seit einigen Jahrtausenden insbesondere in der Schmerztherapie bewährt. Auch in Europa war das Moxakraut im 17. und 18. Jahrhundert ein beliebtes Heilmittel. Es ist allerdings mit dem Aufkommen der « Schulmedizin » in Vergessenheit geraten.

Erwärmen, Anräuchern, Brennen charakterisieren das Wort Moxen wohl am besten. Die heilende Wirkung des Moxens ist nicht so sehr in dem energetischen Anstrahlen zu suchen, sondern vielmehr in einer spezifischen, nur dem Moxakraut eigenen Wirkung begründet. Am verbreitetsten ist das sog. direkte Moxen in der traditionellen chinesichen Medizin, d. h. das Moxakraut wird direkt auf der Haut abgebrannt. Eine sehr wirkungsvolle Therapie, die jedoch für den Patienten auch sehr schmerzhaft sein und häßliche Brandwunden entstehen lassen kann. Diese Art der Moxatherapie wird in der Regel nur von speziell ausgebildeten Therapeuten durchgeführt. Die von Dr. Nottbohm entwickelte Moxatherapie wird dem sog. indirekten Verfahren zugeordnet. Mittels eines von ihm entwickelten Moxers wirkt der heiße Rauch des glimmenden Moxakrautes in vorgegebenen Zeiteinheiten auf festgelegte Hautareale. Der Vorteil des indirekten Moxens liegt darin, daß man die Raucheinwirkung entsprechend der Hitzeverträglichkeit selber steuern kann und somit Brandwunden vermieden werden. Das indirekte Moxen ist einfach in der Anwendung, kann von jedem selbst nach einer Anleitung durchgeführt werden. Es ist das weitaus schonendere Verfahren und steht in der therapeutischen Wirksamkeit der direkten Methode in keiner Weise nach.

**Patentansprüche**

1. Instrument (10) zur indirekten Selbsttherapie bei verschiedenen chronischen Schmerzproblemen, insbesondere Kopf- und Rückenschmerzen, mit einer eine Moxazigarre (8) mittig und verschiebbar aufnehmenden, zylindrischen Rauchkammer (12), die mehrere seitliche Öffnungen (34, 35) aufweist, und bei dem die Rauchkammer auf ihrer an den Körper applizierten Stirnseite eine abnehmbare, mit einer Öffnung (26) versehene Kappe (18) aufweist, deren Öffnung mit einem Schutzgitter (20) versehen ist, dadurch gekennzeichnet, daß in der Rauchkammer an der dem Schutzgitter gegenüberliegenden Stirnseite in einem an die Rauchkammer angrenzenden Massivteil (32) eine mittig angeordnete Öffnung (9) vorgesehen ist, durch die paßgerecht die Moxazigarre in Axialrichtung in die Rauchkammer einschiebbar ist, und daß ein am Massivteil seitlich befestigter Stiel (22) mit Handgriff (30) zur Handhabung des Instruments vorgesehen ist.

2. Instrument nach Anspruch 1, dadurch gekennzeichnet, daß der auf den Körper aufzusetzende Kappenrand (28) wulstartig ausgebildet ist, und nach außen hin kegelförmig verläuft.

3. Instrument nach Anspruch 1 oder 2, dadurch gekennzeichnet, daß ein separater, länglicher Stempel (14), der im Querschnitt etwas kleiner ist als die Öffnung (9) zum Einschieben der Moxazigarre (8) in die Rauchkammer (12) vorgesehen ist, welcher rund ist und am nicht in die Öffnung (9) eingeführten Ende einen Knauf (24) aufweist.

4. Instrument nach einem der Ansprüche 1 bis 3, dadurch gekennzeichnet, daß einige der seitlichen Öffnungen (34) außen einen um sie herum verlaufenden Vorsprung (37) aufweisen.

### Claims

1. Instrument (10) for indirect self-therapy for different chronic pain problems, particularly head and back pains, with a cylindrical smoke chamber (12) centrally and displaceably receiving a moxa cigar (8) and having several lateral openings (34, 35) and in which the face of the smoke chamber applied to the body has a removable cap (18) having an opening (26), provided with a protective grid (20), characterized in that a centrally positioned opening (9) is provided on the face of the smoke chamber opposite to the protective grid on a solid part (32) adjacent to the smoke chamber by means of which the moxa cigar can be axially inserted in precise fitting manner into the smoke chamber and that a post (22) with handle (30) fixed laterally to the solid part is provided for handling the instrument.

2. Instrument according to claim 1, characterized in that the cap edge (28) to be placed on the body has a bead-like construction and passes outwards in a conical manner.

3. Instrument according to claims 1 or 2, characterized in that there is a separate, elongated plunger (14), whose cross-section is somewhat smaller than the opening (9) for inserting the moxa cigar (8) into the smoke chamber (12), said plunger being circular and having a knob (24) on the end not inserted into opening (9).

4. Instrument according to one of the claims 1 to 3, characterized in that certain of the lateral openings (34) are externally provided with a projection (37) passing round the same.

### Revendications

1. Instrument (10) pour l'autothérapie indirecte de différents problèmes douloureux chroniques, en particulier de maux de tête et de dos, avec une chambre à fumée (12) cylindrique, logeant en son centre un cigare de moxa (8) de manière mobile, qui présente plusieurs orifices latéraux (34, 35), dans lequel la chambre à fumée présente sur son côté frontal appliqué sur le corps un capuchon amovible (18) pourvu d'un orifice (26), dont l'orifice est équipé d'une grille protectrice (20), caractérisé en ce qu'un orifice (9) placé centralement est prévu dans la chambre à fumée sur le côté frontal opposé à la grille protectrice dans une pièce massive (32) adjacente à la chambre à fumée, orifice à travers lequel le cigare de moxa peut être introduit par glissement de manière bien adaptée dans la chambre à fumée dans le sens axial et qu'un manche (22) avec une poignée (30), fixé latéralement sur la pièce massive, est prévu pour la manipulation de l'instrument.

2. Instrument selon la revendication 1, caractérisé en ce que le bord du capuchon (28) à poser sur le corps est formé à la manière d'un bourrelet et qu'il est conique vers l'extérieur.

3. Instrument selon la revendication 1 ou 2, caractérisé en ce qu'un poinçon (14) oblong séparé dont la section est un peu inférieure à l'orifice (9) d'introduction du cigare de moxa (8) dans la chambre à fumée (12) est prévu, lequel est rond et présente un pommeau (24) à l'extrémité qui n'est pas introduite dans l'orifice (9).

4. Instrument selon l'une des revendications 1 à 3, caractérisé en ce que plusieurs des orifices latéraux (34) présentent à l'extérieur un rebord (37) qui les entoure.

Fig. 1

# Fig. 2

# Fig.3

# Fig.4

20
28
18
35
34
32
22
9

36
20
18
26
35
34
12
32
22
24

8
17
14
24

2